# EUROPÄISCHE PATENTSCHRIFT

(11) **EP 0 930 902 B1**
(45) Veröffentlichungstag und Bekanntmachung des Hinweises auf die Patenterteilung: **20.11.2002**
(21) Anmeldenummer: 98943819.7
(22) Anmeldetag: 31.07.1998
(51) Int. Cl.: A61M 3/02

(54) **DRUCKBEHÄLTER ZUM BEAUFSCHLAGEN EINES IN DIESEM AUFGENOMMENEN, MIT EINER MEDIZINISCHEN FLÜSSIGKEIT GEFÜLLTEN FLEXIBLEN BEUTELS MIT DRUCK**
PRESSURIZED CONTAINER FOR APPLYING PRESSURE TO A FLEXIBLE BAG FILLED WITH A MEDICAL FLUID AND HOUSED IN THE CONTAINER
CONTENANT SOUS PRESSION POUR LA MISE EN PRESSION D'UNE POCHE SOUPLE REMPLIE D'UN LIQUIDE MEDICAL QUI Y EST LOGEE

(30) Priorität: 01.08.1997 DE 19733278
(43) Veröffentlichungstag der Anmeldung: 28.07.1999
(73) Patentinhaber: Storz-Endoskop GmbH, 8200 Schaffhausen (CH)
(72) Erfinder: HAAN, Harald, CH-8203 Schaffhausen (CH); NOVAK, Pavel, CH-8207 Schaffhausen (CH); SINGER, Stefan, D-78244 Gottmadingen (DE); HÜBSCHER, Roland, CH-8252 Schlatt (CH); BAYER, Andreas, D-78224 Singen (DE)
(74) Vertreter: Weller, Wolfgang, Dr.rer.nat.
(86) Internationale Anmeldenummer: EP9804814
(87) Internationale Veröffentlichungsnummer: WO99006084

(56) Entgegenhaltungen:
- EP-A- 0 852 152
- WO-A-88/07384
- DE-A- 4 137 748
- US-A- 5 207 638

## Beschreibung

Die Erfindung betrifft einen Druckbehälter zum Beaufschlagen eines in diesem aufgenommenen, mit einer medizinischen Flüssigkeit gefüllten flexiblen Beutels mit Druck, um Flüssigkeit aus dem flexiblen Beutel abzugeben, mit einem Gehäuse, das eine Öffnung zum Einbringen des Beutels aufweist, mit einem Deckel zum Abdecken der Öffnung, mit einem Durchbruch zum Abführen einer Flüssigkeitsabführleitung des Beutels aus dem Druckbehälter, und mit einem Verschluß zum dichten Verbinden von Deckel und Gehäuse.

Ein derartiger Druckbehälter ist aus der DE 41 37 748 A1 bekannt.

Bei endoskopischen Untersuchungen oder endoskopischen Eingriffen ist es oftmals notwendig, eine medizinische Flüssigkeit zu Spülzwecken zuzuführen. Dazu bedient man sich der eingangs erwähnten Druckbehälter. Der Beutel mit der darin aufgenommenen medizinischen Flüssigkeit wird samt seiner daran angebrachten Flüssigkeitsabführleitung über die Öffnung in das Gehäuse eingebracht. Die Flüssigkeitsabführleitung, meist in Form eines dünnen Schlauches, muß dann entweder mit einer vom Gehäuse abführenden Leitung verbunden werden, oder durch eine entsprechende Öffnung hindurchgefädelt werden, was umständlich ist und einer hohen Aufmerksamkeit bedarf. Anschließend wird das Gehäuse über den Deckel mittels eines Verschlusses dichtend verschlossen. Bei der eingangs erwähnten DE 41 37 748 A1 verschließt der Deckel das Gehäuse in Art eines Weinfasses, d.h. er wird von innen an die Öffnung angelegt und über einen äußeren verdrehbaren Riegel verriegelt. Das Gehäuse selbst ist als rechteckiger Quader ausgebildet. Bei großen Flüssigkeitsmengen, es sind Beutel mit bis zu 5 l Inhalt in Gebrauch, muß das Gehäuse dann eine dementsprechende Größe aufweisen und muß nicht nur den üblichen Drücken bei der Handhabung standhalten, also etwa 1 - 1,5 bar, sondern muß aufgrund von Zulassungsvorschriften einem Prüfdruck von ca. 4,5 bar widerstehen.

Bei dem eingangs genannten Druckbehälter ist die Handhabung äußerst umständlich, und aufgrund der Quadergeometrie sind entsprechend aufwendig herzustellende Verbindungs- bzw. Schweißnähte längs der Quaderkanten vorgesehen. Insbesondere bei großen Druckbehältern baut die Apparatur sehr sperrig und ist sehr schwer.

Weitere derartige Behälter, die teilweise auch zum Ansaugen von Flüssigkeiten dienen, sind aus der DE 25 36 746 C2, der DE 28 55 270 A1, der DE 35 35 180 A1, der EP 0 040 427 A1 und der US 5 002 534 bekannt.

Es ist daher Aufgabe der vorliegenden Erfindung, einen Druckbehälter der eingangs genannten Art derart weiterzuentwickeln, daß dieser einfach aufgebaut und einfach handhabbar ist und den Druckbedingungen, insbesondere auch den Prüfdruckbedingungen, problemlos standhält.

Erfindungsgemäß wird die Aufgabe dadurch gelöst, daß das Gehäuse als stehender, bodenseitig geschlossener, oben offener hohlzylindrischer Körper ausgebildet ist, dessen Querschnitt flachovalförmig ist und grob der Querschnittskontur eines Beutels entspricht, und daß die beiden gegenüberliegenden großflächigen, stehenden Seitenwände, jeweils spiegelbildlich die Form eines Abschnitts eines Zylindermantels aufweisen, deren seitliche Enden über Verbindungsabschnitte verbunden sind.

Durch die Auswahl eines oben offenen, bodenseitig geschlossenen, stehenden, hohlzylindrischen Körpers ist die Handhabung dadurch vereinfacht, daß der Beutel von oben durch die Öffnung in den stehenden Hohlzylinder eingeschoben werden kann. Aufgrund der Tatsache, daß dessen Querschnitt flachovalförmig ist und grob der Querschnittskontur des Beutels entspricht, resultiert ein Druckbehälter, dessen Form und Größe in etwa dem darin aufzunehmenden Beutel entspricht, so daß keine großen und sperrigen Gerätschaften geschaffen werden müssen. Ein mit Flüssigkeit gefüllter Beutel steht passend im Gehäuse, so daß keine Bereiche des Beutels eingequetscht oder abgeklemmt werden. Aufgrund der Auswahl der Zylindermantelgeometrie halten die Seitenwände, bei relativ dünner Wandstärke, auch einem hohen Prüfdruck von 4,5 bar problemlos Stand, ohne daß Verformungen oder Ausbauchungen zu befürchten sind.

Somit können leichte, schlanke, der Form des Beutels in etwa angepaßte, einfach handhabbare Druckbehälter geschaffen werden. Aufgrund deren Leichtigkeit können die Druckbehälter beispielsweise auch an Stative, die im medizinischen Bereich bei der Zuführung von Flüssigkeiten eingesetzt werden, montiert werden. Nachdem der Beutel einfach durch die ovale obere Öffnung eingeschoben wurde und dann die Flüssigkeitsabführleitung versorgt wurde, braucht der Behälter lediglich noch über den Deckel verschlossen und über den Verschluß dicht verbunden werden. Auch diese Maßnahme ist einfach und sicher auch von ungeschultem Personal durchzuführen. Durch Beaufschlagung mit Druck kann dann die Flüssigkeit aus dem Beutel gezielt, d.h. in der gewünschten Menge pro Zeiteinheit, abgeführt werden.

Somit wird die Aufgabe vollkommen gelöst.

In einer weiteren Ausgestaltung der Erfindung ist der Deckel als ein die ovale obere Öffnung abdeckender und die Oberkante übergreifender Deckel ausgebildet.

Diese Maßnahme hat den Vorteil, daß diese Geometrie ein einfaches und sicheres Anlegen des Deckels an dem Gehäuse nach Einbringen des Beutels eröffnet, so daß auch technisch ungeschultes Personal den Deckel in die zutreffende Schließstellung bringen kann, um dann über den Verschluß die dichte Verbindung herzustellen.

In einer weiteren Ausgestaltung der Erfindung ist zumindest in einer Seitenwand ein Sichtfenster vorgesehen, dessen Scheibe ebenfalls die Form eines Zylindermantelabschnittes aufweist, und die Scheibe ist von der Innenseite des Gehäuses an das Sichtfenster angelegt.

Diese Maßnahme hat nun den Vorteil, daß durch die Auswahl der Geometrie auch bei den Sichtfenstermaterialien einfache und leichte Materialien herangezogen werden, da allein schon durch die Zylindermantelabschnittgeometrie hohen Drücken widerstanden werden kann. Durch Anlegen des Fensters an die Innenseite wird dieses vom Betriebsdruck noch zusätzlich dichtend an die Öffnung angedrückt. Somit können auch großflächige Fenster vorgesehen werden, über die jeweils visuell die Füllstandshöhe des Beutels überprüft werden kann.

In einer weiteren Ausgestaltung der Erfindung ist im Sichtfenster zumindest ein umfänglich oder axial verlaufender Materialsteg des Materials der Seitenwand stehengeblieben.

Diese Maßnahme hat insbesondere bei sehr großen Druckbehältern mit großen Fenstern den Vorteil, daß durch den Materialsteg zum einen die Wand, in der das Fenster vorgesehen ist, stabil bleibt und zum anderen für das an der Innenseite angelegte Fenster eine zusätzliche Abstützstelle vorgesehen ist, so daß beispielsweise Fenstermaterialien aus transparentem Kunststoff herangezogen werden können.

In einer weiteren Ausgestaltung der Erfindung weist der Verschluß zumindest einen an der Außenseite des Gehäuse angeordneten Drehknopf auf, an dessen Innenseite eine Kulissenführung ausgespart ist, in die ein radial vom Deckel vorstehender Kulissenzapfen einführbar ist, und durch Verdrehen des Drehknopfes sperrt die Kulisse dichtend ein Abnehmen des Deckels vom Gehäuse.

Diese Maßnahme hat nun den erheblichen Vorteil, daß durch einfache und betriebssicher zu bewerkstelligende Maßnahmen der Verschluß verschlossen wird. Nur wenn der Drehknopf in der richtigen Stellung befindlich ist, kann der Deckel an das Gehäuse angelegt werden, nämlich dann, wenn der Zapfen überhaupt in die Kulissenführung eintreten kann. Ein einfacher Verdrehvorgang schließt den Verschluß, so daß eine einwandfrei dichtende und fest schließende Verbindung zwischen Deckel und Gehäuse geschaffen ist. Auch dies ist von einem technisch wenig geschulten Personal durchzuführen.

In einer weiteren Ausgestaltung der Erfindung weist der Kulissenzapfen einen vom Deckel radial vorspringenden zylindrischen Schulterabschnitt auf, an den ein mit einer Rolle versehenes Ende folgt, wobei die Kulissenführung derart ausgebildet ist, daß sie in einem ersten Drehabschnitt der Rolle über eine Rampe läuft und erst in der Endstellung ein Endabschnitt mit Anschlag auf dem Schulterabschnitt zum Liegen kommt.

Diese Maßnahme hat nun den erheblichen Vorteil bezüglich der Lebensdauer der Vorrichtung, da beim Verdrehen des Drehknopfes aufgrund der drehbar gelagerten Rolle der Kulissenzapfen im ersten Drehabschnitt reibungs- und widerstandsarm läuft, und erst in der Endstellung steht der Deckel mit dessen zylindrischem Schulterabschnitt unter dem Schließ- und Betriebsdruck stehend mit dem Verschluß in Berührung. Dies erleichtert nicht nur die Handhabung beim Öffnen und Schließen sondern erhöht beachtlich die Lebensdauer des Verschlusses.

In einer weiteren Ausgestaltung der Erfindung ist die Kulissenführung im Bereich des Anschlages hinterschnitten.

Diese Maßnahme hat den Vorteil, daß durch die Hinterschneidung die Handhabungsperson spürbar merkt, daß dieser Bereich erreicht ist. Im Druckbetrieb ist ein versehentliches oder willentliches Öffnen des Verschlusses nicht möglich, da dazu der Kulissenzapfen zunächst aus der Hinterschneidung herausbewegt werden müßte, was mit Handkraft nicht möglich ist. Somit trägt diese Maßnahme erheblich zum Sicherheitsaspekt beim Druckbetrieb bei.

In einer weiteren Ausgestaltung der Erfindung ist zwischen Oberkante des Körpers und dem Deckel eine umlaufende V-Dichtung vorgesehen.

Diese Maßnahme hat den Vorteil, daß dadurch eine Art Selbstabdichtungsmechanismus geschaffen ist, denn mit steigendem Anpreßdruck wird die Abdichtung zwischen Mantel des Gehäuses und Deckel aufgrund der V-Geometrie vergrößert.

In einer weiteren Ausgestaltung der Erfindung sind zwei an den Verbindungsabschnitten der Zylindermantelwände angeordnete, diametral gegenüberliegende Verschlüsse vorgesehen.

Diese Maßnahme hat den Vorteil, daß durch die beiden diametral gegenüberliegenden Verschlüsse der ovale Deckel gleichmäßig verschlossen werden kann.

In einer weiteren Ausgestaltung der Erfindung ist im Deckel zumindest ein Anschluß für die Zuführung eines Druckmediums in den Druckbehälter vorgesehen.

Diese Maßnahme hat den Vorteil, daß solche Druckzuführleitungen die Handhabung beim Einlegen und Entnehmen des Beutels aus dem Gehäuse nicht stören und dann beim Anlegen und Schließen des Deckels zugleich auch automatisch die Zuführung des Druckmediums bereitgestellt wird.

In einer weiteren Ausgestaltung der Erfindung ist die Innenwand des Gehäuses mit einem Gleitbelag versehen.

Diese Maßnahme hat den erheblichen handhabungsmäßigen Vorteil, daß das Einführen des Beutels in den oben offenen, stehenden hohlzylindrischen Körper weiter erleichtert wird. Die Beutel sind ja meist aus Kunststoffmaterialien hergestellt, und das Material des Druckbehälters wird meist Metall sein, somit müssen zwei Materialien aneinander vorbeigleiten, die aufgrund ihrer unterschiedlichen Materialstruktur nicht besonders gut aneinander gleiten. Durch Vorsehen des Gleitbelages, beispielsweise aus einem glatten Teflonmaterial oder dergleichen, gleitet der Beutel sanft in das Gehäuse.

In einer weiteren Ausgestaltung der Erfindung ist der gesamte Druckbehälter an einem Stativ anbringbar.

Wie eingangs erwähnt, ist die einfache und leichte Bauweise ein wesentlicher Vorteil der Erfindung. Dadurch ist es nun möglich, auch relativ große Druckbehälter an in der Medizintechnik üblichen Stativen, meist sogenannten fahrbaren Galgen, anzubringen.

In einer weiteren Ausgestaltung der Erfindung ist das Gehäuse über eine längs der Stativachse aus- und einfahrbare Vorrichtung unverlierbar mit dem Deckel verbunden.

Diese Maßnahme hat den Vorteil, daß bei der Handhabung oder gegebenenfalls einem späteren Reinigungsvorgang nicht versehentlich der Deckel verlegt werden kann, sondern diese beiden wesentlichen Bauelemente unverlierbar miteinander verbunden sind. Durch die längs der Stativachse ein- und ausfahrbare Vorrichtung kann das Gehäuse relativ zum Deckel oder umgekehrt verfahren werden, um beispielsweise einen leeren Beutel zu entnehmen und einen vollen Beutel einzufüllen. Dies erleichtert weiterhin durch einfache Maßnahmen die Handhabung.

In einer weiteren Ausgestaltung der Erfindung ist die Vorrichtung teleskopartig ausgebildet.

Diese Maßnahme hat den Vorteil, daß schlanke, sich längs der Stativachse erstreckende Teleskope eingesetzt werden können, die keinen großen Bauraum benötigen und keine die Handhabung des Druckbehälters störenden sperrigen Bauelemente darstellen.

In einer besonders bevorzugten Ausgestaltung ist der Deckel fest mit dem Stativ verbindbar, und das Gehäuse ist gegenüber dem Deckel längs der Stativachse verfahrbar.

Diese Maßnahme hat nun den erheblichen handhabungsmäßigen Vorteil, daß der Deckel gegebenenfalls mit diversen Anschlüssen ortsfest am Stativ anbringbar ist, und daß zum Einlegen oder Entnehmen eines Beutels das Gehäuse relativ gegenüber dem Dekkel abgesenkt bzw. anschließend wieder angehoben wird.

In einer weiteren Ausgestaltung der Erfindung ist in der Vorrichtung eine Entlastungsfeder vorgesehen, die den Verfahrvorgang des Gehäuses erleichtert.

Diese Maßnahme hat nun den erheblichen handhabungsmäßigen Vorteil, daß durch die Entlastungsfeder diese Anhebe- und Absenkvorgänge ohne Kraftaufwand einfach durchgeführt werden können, also daß auch zierliches Bedienungspersonal solche Wechselvorgänge durchführen kann, da solche Federn über den gesamten Hub eine konstante Kraft ausüben.

In einer weiteren Ausgestaltung der Erfindung ist die Feder eine Gasfeder, eine Rollfeder oder eine Feder mit einer Gedächtnislegierung.

Diese Maßnahme hat den Vorteil, daß über solche Federn der zuvor erwähnte entlastete Absenk- oder Anhebvorgang einfach und sicher durchgeführt werden kann, da solche Federn über den gesamten Hub eine konstante Kraft ausüben.

In einer weiteren Ausgestaltung der Erfindung ist an der Unterseite des Deckels eine Einhängevorrichtung für den Beutel vorgesehen.

Diese Maßnahme hat nun den erheblichen handhabungsmäßigen Vorteil, daß nach Absenken des Gehäuses beispielsweise ein leerer Beutel ausgehängt und ein neuer voller Beutel an die Unterseite angehängt werden kann, was einfach und handhabungsfreundlich durchzuführen ist. Anschließend wird dann das Gehäuse von unten an den Deckel herangeführt, und über die Verschlüsse wird der Druckbehälter verschlossen.

In einer weiteren höchst bevorzugten Ausführung der Erfindung ist in der Oberkante des Gehäuses zumindest eine Ausnehmung vorgesehen, in die von oben die Flüssigkeitsabführleitung des Behälters legbar ist und dadurch aus dem Gehäuse geführt wird.

Diese Maßnahme hat nun den erheblichen handhabungsmäßigen Vorteil, daß die Flüssigkeitsabführleitung des Beutels nicht durch eine Öffnung hindurchgefädelt werden muß oder an einen separaten Anschluß im Gehäuse angeschlossen werden muß, der möglicherweise Quelle einer Kontamination sein könnte. Dieser Einlegevorgang ist einfach durchzuführen, insbesondere mit der zuvor erwähnten Anhebung und Absenkung des Gehäuses. Nach Einhängen des Beutels an die Unterseite wird das Gehäuse von unten an den Deckel herangefahren, wobei die Bedienungsperson die Flüssigkeitsabführleitung lediglich dann in die nach oben offene Ausnehmung einlegen muß, ein Vorgang, der zum einen genau im Blickfeld der Bedienungsperson liegt und auch keine allzu große Aufmerksamkeit erforderlich macht. Bei großen Gehäusen können auch mehrere Ausnehmungen vorgesehen sein, so daß auch zwei oder mehrere Beutel eingebracht und deren Flüssigkeitsabführleitungen jeweils einzeln aus dem Druckbehälter abgeführt werden können.

In einer weiteren Ausgestaltung der Erfindung ist in der Ausnehmung eine Formdichtung einsetzbar, in die die Flüssigkeitsabführleitung einlegbar ist.

Diese Maßnahme hat den Vorteil, daß die Abdichtung sicher gewährleistet ist. Die Formdichtung kann entweder schon an der Flüssigkeitsabführleitung vorgesehen sein oder vor dem Einlegen auf diese aufgeschoben werden.

In einer weiteren Ausgestaltung der Erfindung überdeckt ein die Oberkante des Gehäuses übergreifender Abschnitt des Deckels auch teilweise die Formdichtung.

Diese Maßnahme hat den Vorteil, daß besonders betriebssicher diese Stelle abgedichtet werden kann, da durch den im Innenraum herrschenden Druck die Formdichtung von der Innenseite an den übergreifenden Abschnitt gut dichtend angedrückt wird.

In einer weiteren Ausgestaltung der Erfindung übergreift die Formdichtung die Gehäusewand im Bereich der Ausnehmung innen und außen.

Diese Maßnahme hat den Vorteil, daß eine solche Formdichtung einfach von oben in die Ausnehmung einsteckbar ist und außerdem relativ großflächig um die Ausnehmung anliegt, somit für eine hervorragende Abdichtung sorgt.

In einer weiteren Ausgestaltung der Erfindung ist die Formdichtung geschlitzt, so daß die Flüssigkeitsabführleitung in die Formdichtung eindrückbar ist.

Diese Maßnahme hat den erheblichen handhabungsmäßigen Vorteil, daß das Anbringen der Dichtung an den Schlauch oder umgekehrt vereinfacht durchzuführen ist. Entweder kann die Dichtung auf den Schlauch über den Schlitz aufgedrückt werden, oder zunächst die Dichtung in die Ausnehmung eingesetzt werden und dann der Schlauch der Flüssigkeitsabführleitung eingedrückt werden.

In einer weiteren Ausgestaltung der Erfindung ist am Gehäuse eine Heizung vorgesehen.

Diese Maßnahme hat den erheblichen Vorteil, daß die im Druckbehälter aufgenommenen Beutel warmgehalten oder ggf. erwärmt werden können.

In einer weiteren Ausgestaltung der Erfindung ist am Deckel ein Korb angebracht, in den der Beutel einlegbar ist.

Diese Maßnahme hat den Vorteil, daß die flexiblen Beutel im Korb lagefest gehalten und gestützt werden, so daß insbesondere bei großen Beuteln verhindert werden kann, daß ein großflächiger Kontakt zwischen dem Beutel und der Innenwand des Gehäuses stattfindet. Dadurch wird nicht nur das Einlegen bzw. das Einführen der Beutel in das Innere des Druckbehälters wesentlich erleichtert, sondern es ist auch einfach möglich, einen fälschlicherweise eingebrachten Beutel, der noch prall gefüllt ist, wieder aus dem Druckbehälter zu entnehmen. Insbesondere bei der Ausgestaltung, bei der die Fenster, die die Sichtöffnungen verschließen, von der Innenseite angebracht sind, bestünde die Gefahr, daß sich der Beutel mit den Fenstern verhakt. Wird ein großer Beutel, bspw. mit einem Inhalt von 5 l oder werden zwei Beutel bspw. mit 1,5 l in den Druckbehälter eingesetzt, so ist dies durch den Korb wesentlich erleichtert. Sehr große und schwere Beutel nehmen in abgelegtem Zustand die Form eines sehr breiten Tropfens ein, so daß dann ggf. großflächiger Kontakt mit der Kammerinnenwand stattfinden könnte. Dies wird nun durch den Korb sicher verhindert.

In einer weiteren Ausgestaltung der Erfindung weist der Korb einen Boden auf, der im Abstand über dem geschlossenen Boden des Gehäuses zum Liegen kommt.

Die Flüssigkeitsabführleitung erstreckt sich vom Boden des Beutels weg und muß über eine 180°-Schlaufe vom Boden des Beutels an die obere Kante des Druckbehälters geführt werden. Käme der Boden des Beutels auf dem Boden des Gehäuses des Druckbehälters zum Liegen, könnte die Leitung abknicken und die Abführung der Flüssigkeit sperren. Nunmehr kann der Beutel auf dem Boden des Korbes abgelegt werden und es steht unter dem Boden noch ausreichend Raum zur Verfügung, um die Schlaufe einwandfrei zu führen.

In einer weiteren Ausgestaltung der Erfindung weist der Korb eine Führung für die Flüssigkeitsabführleitung des Beutels auf.

Diese Maßnahme hat nun den erheblichen Vorteil, daß die Flüssigkeitsabführleitung durch die Führung am Korb lagefixiert an das obere Ende des Druckbehälters geführt werden kann, so daß ein Verheddern oder Abklemmen beim Ein- und Ausbringen des Beutels ausgeschlossen ist. Darüber hinaus erleichtert die Führung das Einsenken des Beutels in das Innere des Druckbehälters. Diese genau reproduzierbare Führung der Flüssigkeitsabführleitung stellt auch sicher, daß diese an der Stelle zum Liegen kommt, an der sie aus dem Druckbehälter abgeführt wird, somit ist sichergestellt, daß nicht versehentlich diese Leitung bei Aufsetzen des Deckels abgeklemmt wird.

In einer weiteren Ausgestaltung der Erfindung weist die Führung Haken auf, in die die Flüssigkeitsabführleitung einführbar ist.

Diese Maßnahme hat den Vorteil, daß die Leitung sehr einfach in die Haken eingelegt, eingeschoben oder eingefädelt werden kann und durch entsprechende Formgebung und Ausrichtung der Haken auch fixiert ist.

In einer weiteren Ausgestaltung der Erfindung besteht der Boden aus einem schlangenlinienförmig gewundenen Stabkörper.

Diese Maßnahme hat den erheblichen Vorteil, daß zwischen den schlangenlinienförmigen Windungen der bodenseitige Abgang bzw. Anschlußstutzen des flexiblen Beutels eingelegt werden kann und dort lagefixiert ist. In Zusammenwirken mit der Ausgestaltung, daß dieser Boden im Abstand über dem Boden des Gehäuses zum Liegen kommt, ist sichergestellt, daß unabhängig von der Beutellänge bzw. der Durchhängung des Beutels die Höhenlage des unteren Anschlusses reproduzierbar und fixiert ist, so daß ein Abknicken der abgehenden Abführleitung sicher vermieden werden kann.

In einer weiteren Ausgestaltung der Erfindung ist die Heizung als Wand des Korbes ausgebildet.

Diese Maßnahme hat den Vorteil, daß der in dem Korb eingebrachte Beutel einen maximal möglichen Kontakt mit der Heizung unmittelbar erhält, wodurch der Wirkungsgrad stark verbessert wird. Dadurch, daß nun die Heizung im Inneren des Druckbehälters aufgenommen ist, wird auch der Wirkungsgrad im Hinblick auf eine Wärmeabstrahlung maximiert.

In einer weiteren Ausgestaltung der Erfindung ist vorgesehen, die der Heizung zugehörigen Steuerelemente auf dem Deckel anzubringen.

Diese Maßnahme hat nun den Vorteil, daß die Steuerelemente, die mit der Heizung in Verbindung stehen, an einem Bauteil angebracht sind, nämlich an dem Deckel, an dem auch der Korb angebracht ist, dessen Wand als Heizung ausgebildet ist, so daß eine einfache Handhabung und eine einfache Ausgestaltung dieser Bauelemente möglich ist.

Die Erfindung wird nachfolgend anhand einiger ausgewählter Ausführungsbeispiele im Zusammenhang mit den beiliegenden Zeichnungen näher beschrieben und erläutert. Es zeigen:
- Fig. 1: stark schematisiert ein erstes Ausführungsbeispiel eines erfindungsgemäßen Druckbehälters,
- Fig. 2: eine Seitenansicht eines Gehäuses eines zweiten Ausführungsbeispiels eines erfindungsgemäßen Druckbehälters ohne aufgesetzten Deckel,
- Fig. 3: einen Querschnitt längs der Linie III-III in Fig. 2,
- Fig. 4: eine stark vergrößerte ausschnittsweise Darstellung des Behälters von Fig. 2 im Bereich einer Ausnehmung zur Durchführung der Flüssigkeitsabführleitung mit aufgesetztem Deckel,
- Fig. 5: einen Schnitt längs der Linie V-V in Fig. 4,
- Fig. 6: eine stark vergrößerte ausschnittsweise um 90° gegenüber der Darstellung von Fig. 2 verdrehte Seitenansicht des Gehäuses mit aufgesetztem Deckel, wobei sich der Drehverschluß in seiner Endstellung befindet,
- Fig. 7: einen Schnitt längs der Linie VII-VII in Fig. 6,
- Fig. 8: eine der Darstellung von Fig. 6 vergleichbare Darstellung in einer anderen Betriebsstellung des Verschlusses,
- Fig. 9: eine Seitenansicht des kompletten Druckbehälters der zweiten Ausführungsform an einem Stativ montiert mit abgesenktem Gehäuse,
- Fig. 10: eine der Fig. 9 entsprechende Seitenansicht mit geschlossenem Druckbehälter,
- Fig. 11: eine der Fig. 1 vergleichbare Darstellung eines weiteren Ausführungsbeispiels mit einem am Deckel montierten Korb zur Aufnahme des Beutels, und
- Fig. 12: eine Seitenansicht der Darstellung von Fig. 11.

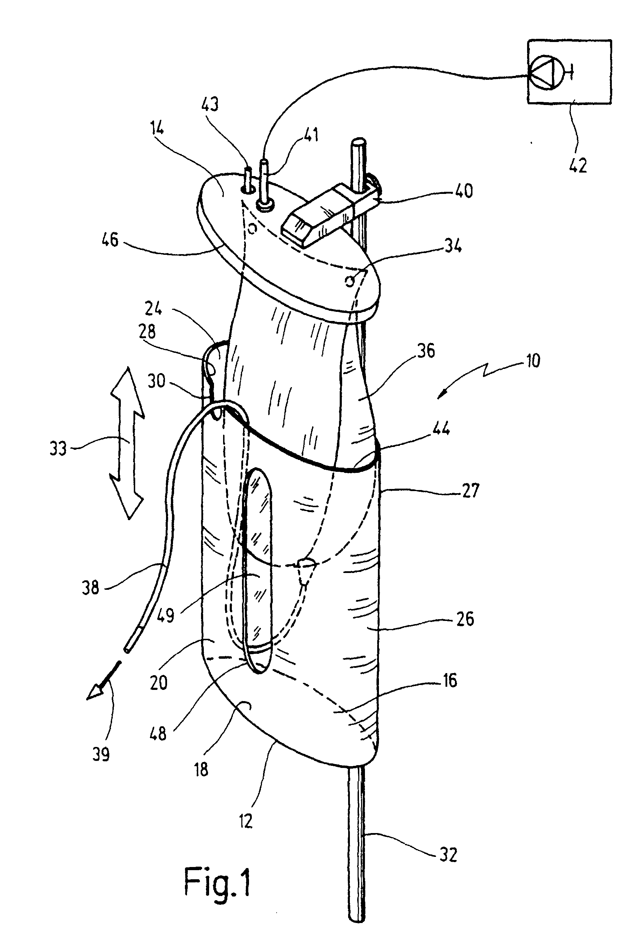

In Fig. 1 ist ein in seiner Gesamtheit mit der Bezugsziffer 10 versehener Druckbehälter dargestellt.

Der Druckbehälter 10 weist ein Gehäuse 12 und einen davon trennbaren Deckel 14 auf.

Das Gehäuse 12 besteht aus einem stehenden, etwa hohlzylindrischen Körper 16, der bodenseitig über einen Boden 18 verschlossen ist.

Das Gehäuse 12 weist zwei gegenüberliegende, spiegelbildlich angeordnete Seitenwände 20, 22 auf, die über Verbindungsabschnitte 24 und 26 miteinander verbunden sind.

Die Seitenwände 20 und 22 haben die Form eines Zylindermantelabschnittes. In einer Oberkante 28 des Gehäuses 12 ist eine Ausnehmung 30 vorgesehen, deren Sinn und Zweck später beschrieben wird.

Der Deckel 14 ist über eine Befestigung 40 an einem Stativ 32 lösbar befestigt. An der Unterseite des Deckels 14 ist eine Einhängevorrichtung 34 vorgesehen, in die ein Beutel 36 eingehängt werden kann.

Der Beutel 36 besteht aus einem flexiblen Kunststoffmaterial und ist mit einer hier nicht näher bezeichneten medizinischen Flüssigkeit gefüllt. Am unteren Ende des Beutels 36 führt eine fest mit diesem verbundene Flüssigkeitsabführleitung 38 ab, und zwar in Form eines am Beutel 36 angeschweißten Schlauches.

Am Deckel 14 ist ein Anschlußstutzen 41 vorhanden, der über eine Leitung mit einer Druckquelle 42 verbindbar ist. Ferner ist am Deckel ein Überdruckventil 43 vorgesehen.

Aus der perspektivischen Darstellung ist zu erkennen, daß der oben offene hohlzylindrische Körper 16 im Querschnitt die Form eines Flachovales aufweist, somit am oberen Ende eine ovale Öffnung 44 resultiert, die grob der Querschnittskontur des Beutels 36 entspricht, so daß dieser passend in der durch den Körper 16 umgrenzten Kammer aufnehmbar ist.

In der Seitenwand 20 ist ein Fenster 48 vorgesehen, das von einer innen angelegten Scheibe 49 verschlossen ist.

Am Gehäuse 12 ist eine hier nicht dargestellte Heizung in Form einer flächigen Heizmatte vorgesehen, um die im Beutel 36 enthaltene Flüssigkeit entweder warmzuhalten (z.B. auf 37° oder 42°C) oder ggf. zu erwärmen.

Die Handhabung des Druckbehälters 10 ist wie folgt. Zum Einlegen eines mit einer Flüssigkeit gefüllten Beutels 36 wird das Gehäuse 12 abgesenkt, d.h. längs des Statives 32 nach unten verschoben. Anschließend wird an die Unterseite des Deckels 14 der Beutel 36 gehängt. Danach wird das Gehäuse 12 von unten über den am Deckel 14 hängenden Beutel 36 geschoben. Die Handhabungsperson erfaßt die Flüssigkeitsabführleitung 38 mit einer Hand und sorgt dafür, daß diese von oben in der Ausnehmung 30 zum Liegen kommt. Das Gehäuse 12 wird soweit angeschoben, bis dessen Oberkante 28 an der Unterseite des Deckels 14 zum Liegen kommt. Dazu wird eine hier nicht dargestellte ovalförmige V-Dichtung zwischen Gehäuse 12 und Deckel 14 gelegt. Der Deckel 14 weist einen nach unten vorspringenden, übergreifenden Abschnitt 46 auf, der so ausgebildet ist, daß er auch teilweise die Ausnehmung 30 überdeckt, so daß dann ein dichtes Abführen der Flüssigkeitsabführleitung 38 bewerkstelligt werden kann, wie das später im Zusammenhang mit anderen Ausführungsbeispielen noch näher beschrieben werden wird. Nachdem das Gehäuse 12 entsprechend verschoben wurde, wie das ja durch den Doppelpfeil 33 angezeigt ist, wird über einen hier nicht dargestellten Verschluß der Deckel 14 fest mit dem Gehäuse 12 verschlossen.

Über die Druckquelle 42 kann nun ein gasförmiges Druckmedium, z.B. Druckluft oder CO₂ in das Innere, also in die Druckkammer des Druckbehälters 10 eingeführt werden und nach und nach ein Druck aufgebaut werden. Dieser Druck sorgt dafür, daß der Beutel 36 zusammengequetscht und dessen darin enthaltene Flüssigkeit über die Flüssigkeitsabführleitung 38 aus dem Druckbehälter 10 abgeführt wird, wie das durch einen Pfeil 39 angedeutet ist. Den jeweiligen Füllstand des Beutels 36 kann man über das Fenster 48 verfolgen. Ist der Beutel 36 entleert oder der Spülvorgang beendet, wird der Innenraum auf Atmosphärendruck entlüftet, der (nicht dargestellte) Verschluß geöffnet, und das Gehäuse 12 kann wieder abgesenkt werden, so daß der nunmehr leere Beutel 36 entnommen und ein neuer eingesetzt werden kann.

Durch die Durchführung der Flüssigkeitsabführleitung 38 über die Ausnehmung 30 muß die Flüssigkeitsabführleitung 38 nicht durch ein Loch, beispielsweise im Boden, durchgefädelt werden, so daß dann die vom Hersteller vorgesehene Flüssigkeitsabführleitung 38 direkt, ohne Zwischenschaltung einer Anschlußleitung oder Durchführleitung, beispielsweise an das Instrument, über das die Spülflüssigkeit zugeführt werden soll, angeschlossen werden kann.

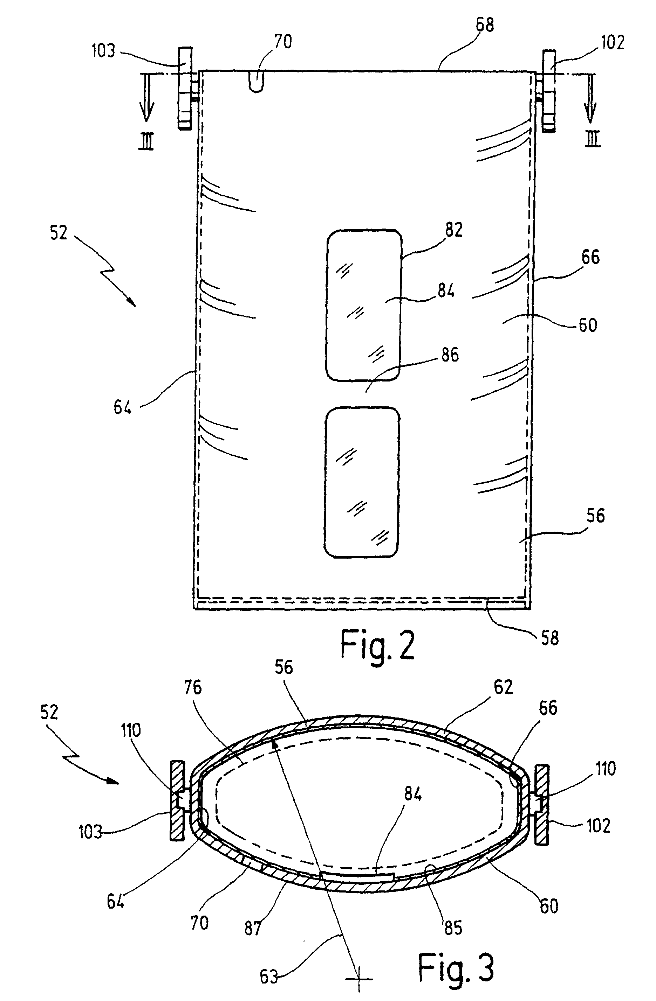

Im Zusammenhang mit den Figuren 2 bis 10 wird die Ausgestaltung des Verschlusses und die druckdichte Abführung der Flüssigkeitsabführleitung näher beschrieben und erläutert.

Der in den Figuren 2 bis 10 dargestellte Druckbehälter ist in seiner Gesamtheit mit der Bezugsziffer 50 versehen.

Der Druckbehälter 50 weist ebenfalls, wie zuvor beschrieben, ein Gehäuse 52 und einen Deckel 54 auf.

Das Gehäuse 52 weist, wie das insbesondere aus den Darstellungen von Fig. 2 und 3 ersichtlich ist, einen stehenden, am unteren Ende über einen Boden 58 verschlossenen, oben offenen hohlzylindrischen Körper 56 auf.

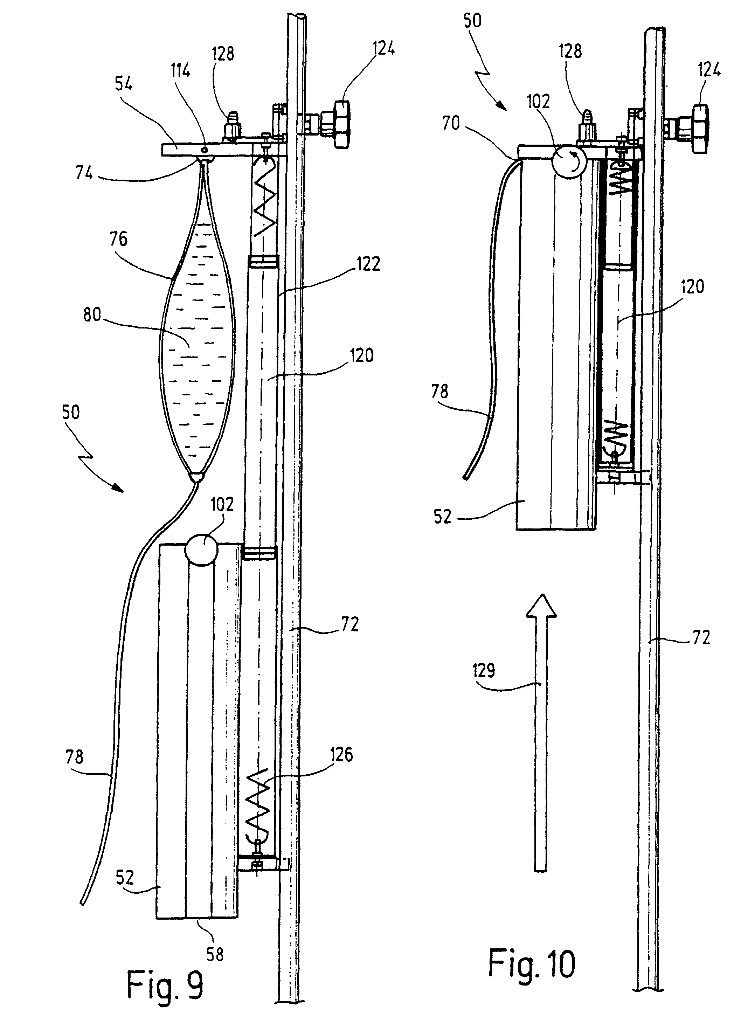

Der Körper 56 weist zwei spiegelbildlich gegenüberstehende, nach außen gewölbte Seitenwände 60 und 62 auf, die über abgeflachte Verbindungsabschnitte 64 und 66 miteinander verbunden sind. In Fig. 3 ist durch einen Radius 63 angedeutet, daß die Seitenwand 62 als Abschnitt eines Zylindermantels mit dem Radius 63 ausgebildet ist. Dasselbe gilt für die Seitenwand 60. In der Oberkante 68 ist, wie zuvor beschrieben, eine Ausnehmung 70 vorgesehen, die zur Durchführung der Flüssigkeitsabführleitung 78 dient. Aus Fig. 9 ist zu entnehmen, daß der Deckel 54 über eine Befestigung 124 an einem Stativ 72 angebracht ist.

An der Unterseite des Deckels 54 ist ebenfalls eine Einhängevorrichtung 74 vorgesehen, an die ein Beutel 76 angehängt werden kann. Bodenseitig führt vom Beutel 76 eine Flüssigkeitsabführleitung 78 in Form eines Schlauches ab. Im Beutel 76 aus einem transparenten Kunststoffmaterial ist eine medizinische Flüssigkeit 80 aufgenommen. Im Anschlußbereich der Flüssigkeitsabführleitung 78 kann ein Druckventil angeordnet sein, so daß, beispielsweise in der in Fig. 9 dargestellten Stellung, über die Schwerkraft keine Flüssigkeit abfließt, sondern daß dazu ein gewisser Preßdruck überschritten werden muß.

Zurückkehrend zu Fig. 2 ist dort ersichtlich, daß in der Seitenwand 60 ein Fenster 82 vorgesehen ist, das über eine transparente Scheibe 84 verschlossen ist.

Wie aus der Schnittdarstellung von Fig. 3 zu erkennen, ist die Scheibe 84 an die Innenwand 85 im Bereich des Fensters 82 angelegt und weist ebenfalls die Form eines Zylindermantelabschnittes auf. Das Fenster 82 ist über einen horizontal verlaufenden Materialsteg 86 in zwei etwa gleichgroße Abschnitte aufgeteilt. Der Steg 86 erhöht nicht nur die Stabilität der Seitenwand 60 sondern stellt auch eine zusätzliche Abstützung der Scheibe 84 gegen Ausbauchen oder Auswölben dar.

Die Innenwand 85 des gesamten Gehäuses 52 ist mit einem Gleitbelag 87 versehen, beispielsweise einer Beschichtung mit Teflon.

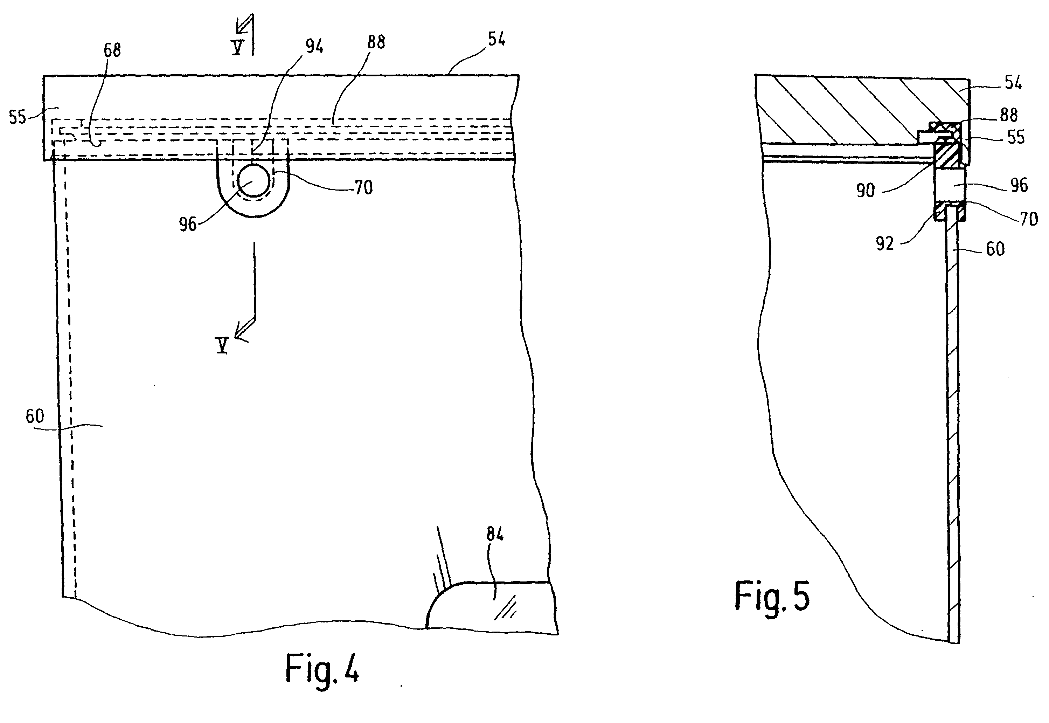

Auf der Oberkante 68 liegt eine umlaufende V-Dichtung 88, wie das insbesondere aus den vergrößerten Schnittdarstellungen von Fig. 4 und 5 ersichtlich ist.

Die V-Dichtung 88 überdeckt somit auch die Ausnehmung 70 in der Oberkante 68.

In die Ausnehmung 70 ist zusätzlich eine Formdichtung 90 eingelegt, wie das ebenfalls aus den Schnittdarstellungen von Fig. 4 und 5 ersichtlich ist.

Die Formdichtung 90 weist einen im wesentlichen U-förmigen Körper auf, der von oben in die Ausnehmung 70 eingesetzt werden kann. Die Formdichtung 90 weist beidseits die Seitenwand 60 übergreifende Bereiche 92 auf, so daß diese sowohl innen als auch außen die die Ausnehmung 70 umgrenzenden Wandbereiche überdeckt. Am oberen Ende erstreckt sich die Formdichtung 90 bis an die Unterseite der V-Dichtung 88. Wird der Deckel 54 aufgelegt, so übergreift bzw. überdeckt dessen nach unten gerichteter übergreifender Abschnitt 55 den oberen Bereich der Formdichtung 90 und erstreckt sich bis nahe an dessen mittige Öffnung 96 heran, durch die die Flüssigkeitsabführleitung 78 des Beutels 76 dichtend hindurchgeführt wird.

Je nach Ausgestaltung kann die Formdichtung 90 bereits herstellerseitig auf die Flüssigkeitsabführleitung 78 geschoben sein, es ist auch möglich, dies vor Ort zu tun. Eine weitere Erleichterung ist dadurch geschaffen, daß die Formdichtung 90 am oberen Bereich mit einem durchgehenden Schlitz 94 versehen ist, so daß von oben der Schlauch der Flüssigkeitsabführleitung 78 eingedrückt werden kann. Im Druckbetrieb ist ja die Formdichtung 90, wie das insbesondere aus den Darstellungen von Fig. 4 und 5 ersichtlich ist, dann im geschlitzten Bereich durch den übergreifenden Abschnitt 55 des Deckels 54 bedeckt, so daß der Anpreßdruck für eine zusätzlich dichtende Wirkung sorgt, die auch noch durch die Spreizung der V-Dichtung 88 zusätzlich gesichert wird.

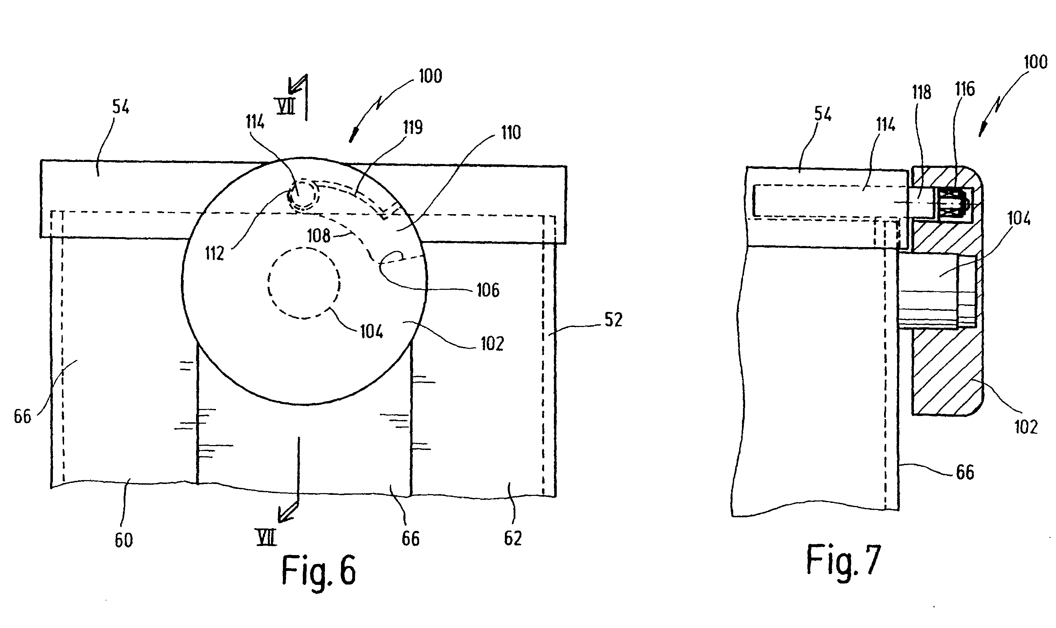

In den Figuren 6 bis 8 sind die Verschlüsse 100 zum Verschließen des Druckbehälters 50 näher dargestellt.

Jeder Verschluß 100 weist an den diametral gegenüberliegenden Verbindungsabschnitten 64 bzw. 66 des Gehäuses 52 über radial vorspringende Achszapfen 104 drehbar an der Außenseite angebrachte Drehknöpfe 102 bzw. 103 auf.

An der Innenseite jedes Drehknopfes 102 bzw. 103 ist eine Kulissenführung 106 in Form einer darin eingeschnittenen Nut 108 ausgespart.

Die Nut 108 weist eine radial mündende Öffnung 110 auf, über die ein radial vom übergreifenden Abschnitt 55 des Deckels 54 nach außen vorspringender Kulissenzapfen 114 eintreten kann.

Wie insbesondere aus Fig. 7 ersichtlich, weist der Kulissenzapfen 114 ein Ende auf, auf dem drehbar eine Rolle 116 bzw. ein Nadellager montiert ist, weiter innen liegt ein massiver Schulterabschnitt 118.

Aus der Schnittdarstellung von Fig. 6 ist zu erkennen, daß die sich etwa in Richtung des Umfangs des Drehknopfes 102 erstreckende Nut 108 eine Rampe 119 aufweist, die so ausgebildet ist, daß, nachdem der Kulissenzapfen 114 über die Öffnung 110 in die Nut 108 eingetreten ist und der Drehknopf 102 verdreht wird, der Kulissenzapfen 114 zunächst über die Rolle 116 an einer Flanke der Nut 108 abrollt. Erst wenn der Drehknopf 102 so weit verdreht ist, daß der Kulissenzapfen 114 einen Anschlag 112 erreicht hat, liegt der Kulissenzapfen 114 mit dessen Schulterabschnitt 118 an der Flanke der Nut 108 an, wie das aus Fig. 7 ersichtlich ist. Die Rolle 116 liegt dabei nicht mehr an einer Flanke der Nut 108 an.

Diese Ausgestaltung erleichtert die Handhabung bzw. die Drehbewegung des Drehknopfes 102 beim Schließen des Verschlusses 100, die durch die Abrollbewegung der Rolle 116 erleichtert wird. Erst in der Endstellung ruht der Schulterabschnitt 118 formschlüssig im Anschlag 112, durch die massive Ausbildung des Schulterabschnittes 118 kann den im Druckbetrieb auf den Verschluß 100 einwirkenden Kräften gut widerstanden werden.

Zum Ansetzen des Deckels 54 an das Gehäuse 52 werden also die Drehknöpfe 102 so verdreht, daß die Öffnungen 110 der Nut 108 nach oben weisen, wie das aus Fig. 3 ersichtlich ist. Durch Aufsetzen des Deckels 54 können nun dessen radial vorspringende Kulissenzapfen 114 in die Nut 108 über deren Öffnung 110 eintreten. Durch Verdrehen der Drehknöpfe 102 wird dann der Kulissenzapfen 114 bis an den Anschlag 112 gebracht.

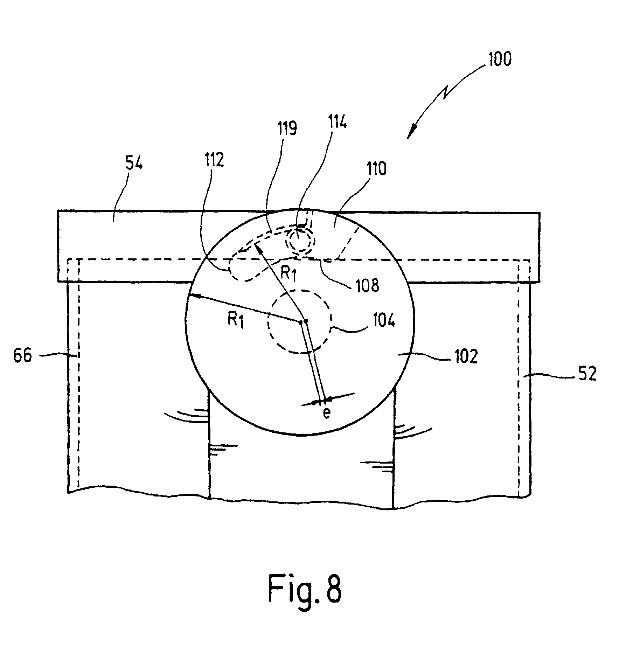

Aus Fig. 8 ist ersichtlich, daß die Bahn der Rampe 119 zwar kreislinienförmig ist, aber aufgrund der Exzentrizität e nach und nach die notwendige Verschlußkraft aufgebracht wird. Hat der Schulterabschnitt 118 den Anschlag 112 erreicht, sitzt dieser fest in einer Hinterschneidung bzw. einer Art Mulde, so daß dadurch schon eine gewisse Selbsthaltekraft oder Vorspannkraft geschaffen ist, so daß auch durch versehentliches Anstoßen an die Drehknöpfe 102 der Verschluß 100 nicht geöffnet werden kann. Die Handhabungsperson stellt den Einrastvorgang in die Hinterschneidung spürbar fest. Diese Vorhaltekraft sorgt auch dafür, daß, wie zuvor beschrieben, die Formdichtung 90 und die V-Dichtung 88 schon dichtend aneinanderliegen, so daß eine gasdichte Durchführung der Flüssigkeitsabführleitung 78 aus dem Druckbehälter 50 gewährleistet ist. Im Druckbetrieb stellt die Hinterschneidung sicher, daß der Verschluß 100 weder versehentlich noch absichtlich geöffnet werden kann, da dazu der Deckel 54 gegen die Schließkraft bewegt werden müßte, was nicht mit Handkraft möglich ist.

Aus den Figuren 9 und 10 ist zu entnehmen, daß der am Stativ 72 angebrachte Deckel 54 über eine Vorrichtung 120 mit dem Gehäuse 52 verbunden ist.

Die Vorrichtung 120 weist ein Dreifachteleskop 122 auf, in dessen Innerem eine Entlastungsfader 126 angeordnet ist. Die Entlastungsfeder 126 ist so ausgestaltet, daß das Gewicht des Gehäuses 52 kompensiert wird, d.h. es kann zum einen sehr einfach und in jeder Höhenstellung verbleibend längs des Statives 72 verschoben werden.

In der Darstellung von Fig. 9 ist das Gehäuse 52 so weit abgesenkt, daß an die Einhängevorrichtung 74 an der Unterseite des Deckels 54 ein Beutel 76 angehängt werden kann. Anschließend wird das Gehäuse 52 angehoben, wie das in Fig. 10 durch einen Pfeil 129 angedeutet ist. Es muß nun dafür Sorge getragen werden, daß die Flüssigkeitsabführleitung 78 in die Ausnehmung 70 in der Oberkante eingelegt wird bzw. in die darin aufgenommene Formdichtung 90 eingedrückt wird. Das Gehäuse 52 wird so weit angehoben, bis die radial vorstehenden Kulissenzapfen 114 des Deckels 54 in die Öffnung 110 der Nut 108 der Kulissenführung 106 eingetreten sind. Anschließend werden die Drehknöpfe 102 verdreht, wie das in Fig. 10 durch einen Pfeil 129 bei dem Drehknopf 102 angedeutet ist. Dadurch wird der Verschluß 100 geschlossen, also der Deckel 54 gasdicht mit dem Gehäuse 52 verbunden, die Flüssigkeitsabführleitung 78 ragt aus dem Druckbehälter 50 vor und kann dann an entsprechende Gerätschaften angeschlossen werden.

Über den Anschluß 128 wird dann im Betrieb das Druckmedium in die innere Druckkammer des Druckbehälters 50 eingeführt und dadurch die Flüssigkeit 80 aus dem Beutel 76 herausgedrückt bzw. über die Flüssigkeitsabführleitung 78 abgeleitet.

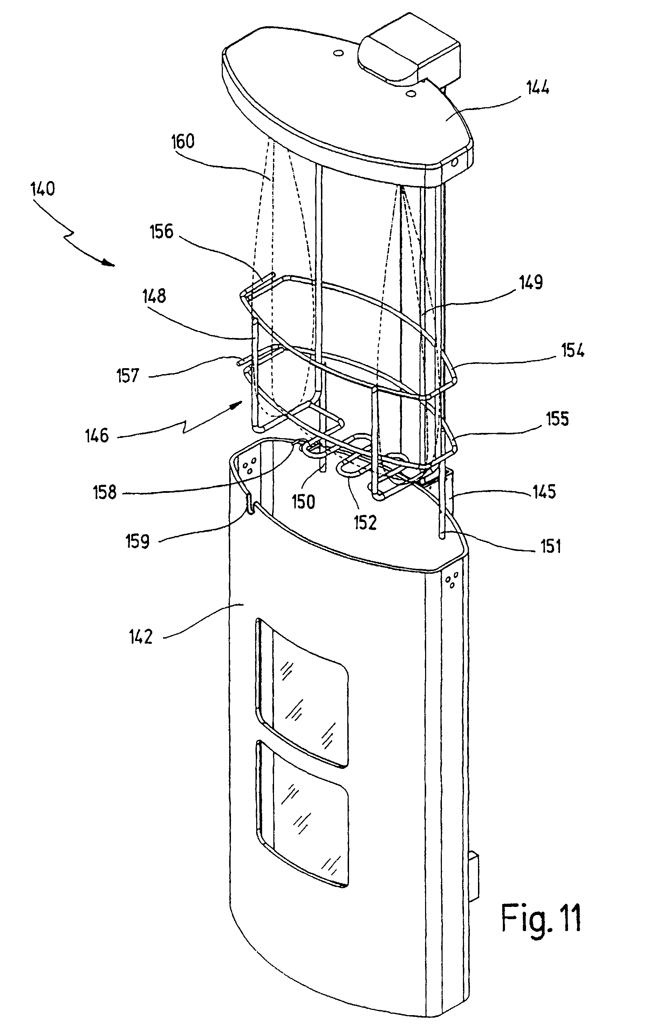

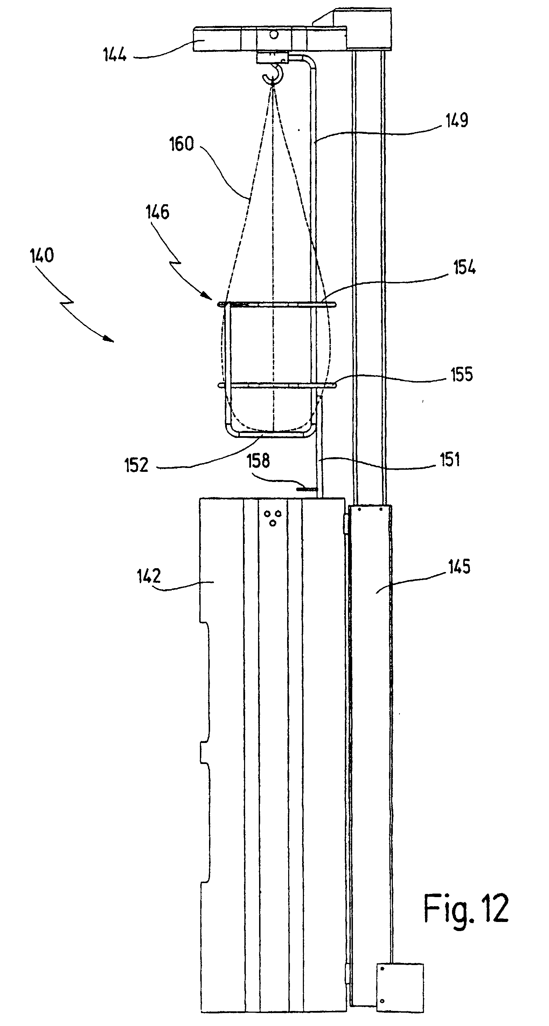

Bei dem in Fig. 11 und 12 gezeigten weiteren Ausführungsbeispiel eines Druckbehälters 140 weist dieser ein Gehäuse 142 auf, das ähnlich wie das in Zusammenhang mit den Fig. 2 bis 10 beschriebene Ausführungsbeispiel mit zwei hier nicht näher bezeichneten Fenstern versehen ist.

Ein Deckel 144 ist über eine teleskopartig verschiebbare Vorrichtung 155 relativ zum Gehäuse anhebbar bzw. absenkbar, wie das zuvor in Zusammenhang mit der Ausführung von Fig. 9 und 10 beschrieben worden ist. Die Verschlüsse sind der Übersicht halber nicht dargestellt.

An der Unterseite des Deckels 144 ist ein Korb 146 montiert, der zur Aufnahme eines oder mehrerer Beutel 160 vorgesehen ist.

Der Korb 146 weist zwei Seitenbügel 148, 149 auf, deren unterer Endbereich U-förmig gebogen ist.

Vom unteren Ende der Seitenbügel 158, 159 steht jeweils ein Stab 150, 151 vor, die als Distanzhalter bzw. Stützfüße des Korbes 146 bezüglich des geschlossenen Bodens des Gehäuses 142 dienen.

Die beiden Seitenbügel 148, 149 sind über zwei Umrandungen 154, 155 miteinander verbunden.

Die Kontur der Umrandungen 154, 155 entspricht in etwa der Querschnittskontur des Gehäuses 142 und diese sind so bemessen, daß sie mit Abstand zur Innenwand des Gehäuses 142 zum Liegen kommt.

Die Umrandungen 154, 155 bestehen jeweils aus einem zu einem Oval gebogenen Stab. Die äußeren Enden der gebogenen Stäbe übergreifen bzw. überlappen seitlich einander, so daß dadurch Haken 156 bzw. 157 gebildet werden, in die seitlich die hier der Übersichtlichkeit halber nicht dargestellte Flüssigkeitsabführleitung, in Form eines Schlauches, wie er in Fig. 1 gezeigt ist, seitlich eingeschoben werden kann.

Der Boden 152 des Korbes 146 ist als schlangenlinienförmig bzw. mäanderlinienförmiger gewundener Stab ausgebildet, wodurch nischenartige Bereiche gebildet werden, in die ein nach unten abstehender Anschlußstutzen des Beutels 160 eingelegt werden kann, wie er bspw. bei dem Beutel 36 in Fig. 1 dargestellt ist.

Wie aus Fig. 1 zu entnehmen, muß die vom Boden des Beutels abgehende Schlauchleitung über eine Schlaufe um 180° gebogen nach oben geführt werden. Diese Führung wird nun durch die zuvor beschriebenen Haken 156 und 157 sowie durch einen weiteren Haken 158, der am Stab 150 vorgesehen ist, bewerkstelligt.

Wie aus der Darstellung von Fig. 12 zu entnehmen, ist der Beutel 160 durch den Korb 146 so gehalten, daß kein großflächiger Kontakt mit der Innenwand des Gehäuses 142 stattfinden kann. Der vom Boden des Beutels 160 nach unten vorstehende Anschlußstutzen (hier der Übersichtlichkeit halber nicht dargestellt) kann zwischen die Schlangenlinienförmigen Windungen des Bodens 152 gelegt werden. Es steht unterhalb des Bodens 152 ausreichend Platz zur Verfügung, um den abgehenden Schlauch um 180° in Form einer Schlaufe nach oben abzuführen. Dieser wird dann in die Haken 158, 157, 156 eingefädelt bzw. eingeschoben und damit sicher und lagefixiert zur Ausnehmung 159 am oberen Ende des Gehäuses 142 geführt, über die dann die Leitung aus dem Gehäuse abgeführt wird. Der im Korb 146 aufgenommene Beutel 160 ist außerdem in einem Haken an der Unterseite des Deckels 144 eingehängt.

Ist eine Heizung vorgesehen, kann diese als eine Wand des Korbes ausgeführt werden, so daß dann ein in den Korb eingesetzter Beutel großflächig mit der Heizung in Berührung steht.

## Patentansprüche

1. Druckbehälter zum Beaufschlagen eines in diesem aufgenommenen, mit einer medizinischen Flüssigkeit (80) gefüllten flexiblen Beutels (36, 76, 160) mit Druck, um die Flüssigkeit (80) aus dem flexiblen Beutel (36, 76, 160) abzugeben, mit einem Gehäuse (12, 52, 142), das eine Öffnung (44) zum Einbringen des Beutels (36, 76, 160) aufweist, mit einem Deckel (14, 54, 144) zum Abdecken der Öffnung (44), mit einem Durchbruch zum Abführen einer Flüssigkeitsabführleitung (38, 78) des Beutels (36, 76, 160) aus dem Druckbehälter (10, 50, 140), und mit einem Verschluß (100) zum dichten Verbinden von Deckel (14, 54, 144) und Gehäuse (12, 52, 142), **dadurch gekennzeichnet, daß** das Gehäuse (12, 52, 142) als stehender, bodenseitig geschlossener, oben offener hohlzylindrischer Körper (16, 56) ausgebildet ist, dessen Querschnitt flachovalförmig ist, und der grob der Querschnittskontur eines Beutels (36, 76, 160) entspricht, und daß die beiden gegenüberliegenden großflächigen stehenden Seitenwände (20, 22, 60, 62), jeweils spiegelbildlich, die Form eines Abschnitts eines Zylindermantels aufweisen, deren seitliche Enden über Verbindungsabschnitte (24, 26; 64, 66) verbunden sind.

2. Druckbehälter nach Anspruch 1, **dadurch gekennzeichnet, daß** der Deckel (14, 54, 144) als die ovale obere Öffnung (44) abdeckender und die Oberkante (28, 68) des Körpers (16, 56) übergreifender Deckel (14, 54, 144) ausgebildet ist.

3. Druckbehälter nach Anspruch 1 oder 2, **dadurch gekennzeichnet, daß** in zumindest einer Seitenwand (20, 60) ein Sichtfenster (48, 82) vorgesehen ist, dessen Scheibe (49, 84) ebenfalls die Form eines Zylindermantelabschnittes aufweist, und daß die Scheibe (49, 84) von der Innenseite (85) des Gehäuses (52) an das Sichtfenster (48, 82) angelegt ist.

4. Druckbehälter nach Anspruch 3, **dadurch gekennzeichnet, daß** im Sichtfenster (82) zumindest ein umfänglich oder axial verlaufender Materialsteg (86) des Materials der Seitenwand (60) stehengeblieben ist.

5. Druckbehälter nach einem der Ansprüche 1 bis 4, **dadurch gekennzeichnet, daß** der Verschluß (100) zumindest einen an der Außenseite des Gehäuses (52) angeordneten Drehknopf (102) aufweist, an dessen Innenseite eine Kulissenführung (106) ausgespart ist, in die ein radial vom Deckel (54) vorstehender Kulissenzapfen (114) einführbar ist, und daß durch Verdrehen des Drehknopfes (102) die Kulisse ein Abnehmen des Deckels (54) vom Gehäuse (52) dichtend sperrt.

6. Druckbehälter nach Anspruch 5, **dadurch gekennzeichnet, daß** der Kulissenzapfen (114) einen von dem Deckel (54) radial vorspringenden zylindrischen Schulterabschnitt (118) aufweist, an den ein mit einer Rolle (116) oder einem Nadellager versehenes Ende folgt, wobei die Kulissenführung (106) derart ausgestaltet ist, daß in einem ersten Drehabschnitt die Rolle (116) oder das Nadellager über eine Rampe (119) läuft, und erst in der Endstellung ein Endabschnitt der Kulissenführung (106) mit Anschlag (112) auf dem Schulterabschnitt (118) des Kulissenzapfens (114) zum Liegen kommt.

7. Druckbehälter nach Anspruch 5 oder 6, **dadurch gekennzeichnet, daß** die Kulissenführung (106) im Bereich des Anschlages (112) hinterschnitten ist.

8. Druckbehälter nach einem der Ansprüche 1 bis 7, **dadurch gekennzeichnet, daß** zwischen Oberkante (68) des Körpers (56) und dem Deckel (14) eine umlaufende V-Dichtung (88) vorgesehen ist.

9. Druckbehälter nach einem der Ansprüche 1 bis 8, **dadurch gekennzeichnet, daß** zwei an den Verbindungsabschnitten (64, 66) der Zylindermantelwände angeordnete, diametral gegenüberliegende Verschlüsse (100) vorgesehen sind.

10. Druckbehälter nach einem der Ansprüche 1 bis 9, **dadurch gekennzeichnet, daß** im Deckel (14, 54) zumindest ein Anschluß (41, 128) für die Zuführung eines Druckmediums in den Druckbehälter (10, 50) vorgesehen ist.

11. Druckbehälter nach einem der Ansprüche 1 bis 10, **dadurch gekennzeichnet, daß** die Innenwand (85) des Gehäuses (12, 52) mit einem Gleitbelag (87) versehen ist.

12. Druckbehälter nach einem der Ansprüche 1 bis 11, **dadurch gekennzeichnet, daß** der Druckbehälter (10, 50) an einem Stativ (32, 72) anbringbar ist.

13. Druckbehälter nach Anspruch 12, **dadurch gekennzeichnet, daß** das Gehäuse (12, 52, 142) über eine längs der Stativachse aus- und einfahrbare Vorrichtung (120, 145) unverlierbar mit dem Deckel (54, 144) verbunden ist.

14. Druckbehälter nach Anspruch 13, **dadurch gekennzeichnet, daß** die Vorrichtung (120, 145) teleskopartig ausgebildet ist.

15. Druckbehälter nach einem der Ansprüche 12 bis 14, **dadurch gekennzeichnet, daß** der Deckel (14, 54) fest mit dem Stativ (32, 72) verbindbar ist, und daß das Gehäuse (12, 52) gegenüber dem Deckel (14, 54) längs der Stativachse verfahrbar ist.

16. Druckbehälter nach einem der Ansprüche 13 bis 15, **dadurch gekennzeichnet, daß** in der Vorrichtung (120) eine Entlastungsfeder (126) vorgesehen ist, die den Verfahrvorgang des Gehäuses (12, 54) erleichtert.

17. Druckbehälter nach Anspruch 16, **dadurch gekennzeichnet, daß** die Entlastungsfeder (126) aus einer Gasfeder, einer Rollfeder oder einer Feder aus einer Gedächtnislegierung besteht.

18. Druckbehälter nach einem der Ansprüche 1 bis 17, **dadurch gekennzeichnet, daß** an der Unterseite des Deckels (14, 54, 144) eine Einhängevorrichtung (34, 74) für einen Beutel (36, 76, 160) vorgesehen ist.

19. Druckbehälter nach einem der Ansprüche 1 bis 18, **dadurch gekennzeichnet, daß** in der Oberkante (28, 68) des Gehäuses (12, 52, 142) zumindest eine Ausnehmung (30, 70, 159) vorgesehen ist, in die von oben die Flüssigkeitsabführleitung (38, 78) des Beutels (36, 76, 160) legbar ist, und dadurch aus dem Gehäuse (12, 52, 142) geführt wird.

20. Druckbehälter nach Anspruch 19, **dadurch gekennzeichnet, daß** in der zumindest einen Ausnehmung (30, 70) eine Formdichtung (90) einsetzbar ist, in die die Flüssigkeitsabführleitung (38, 78) einlegbar ist.

21. Druckbehälter nach Anspruch 20, **dadurch gekennzeichnet, daß** ein die Oberkante (28, 68) des Gehäuse (12, 52) übergreifender Abschnitt (55) des Deckels (52) auch teilweise die Formdichtung (90) überdeckt.

22. Druckbehälter nach Anspruch 20 oder 21, **dadurch gekennzeichnet, daß** die Formdichtung (90) die Gehäusewand (60) im Bereich der Ausnehmung (70) innen und außen übergreift.

23. Druckbehälter nach einem der Ansprüche 20 bis 22, **dadurch gekennzeichnet, daß** die Formdichtung (90) geschlitzt ist, so daß die Flüssigkeitsabführleitung (78) in die Formdichtung (90) eindrückbar ist.

24. Druckbehälter nach einem der Ansprüche 1 bis 23, **dadurch gekennzeichnet, daß** am Gehäuse (12, 52) eine Heizung vorgesehen ist.

25. Druckbehälter nach einem der Ansprüche 1 bis 24, **dadurch gekennzeichnet, daß** am Deckel (144) ein Korb (146) angebracht ist, in den der Beutel (160) einlegbar ist.

26. Druckbehälter nach Anspruch 25, **dadurch gekennzeichnet, daß** der Korb (146) einen Boden (152) aufweist, der im Abstand über dem geschlossenen Boden des Gehäuses (142) zum Liegen kommt.

27. Druckbehälter nach Anspruch 25 oder 26, **dadurch gekennzeichnet, daß** der Korb (146) eine Führung für die Flüssigkeitsabführleitung des Beutels (160) aufweist.

28. Druckbehälter nach Anspruch 27, **dadurch gekennzeichnet, daß** die Führung für die Flüssigkeitsabführleitung Haken (156, 157, 158) aufweist, in die die Flüssigkeitsabführleitung einführbar ist.

29. Druckbehälter nach einem der Ansprüche 26 bis 28, **dadurch gekennzeichnet, daß** der Boden (160) aus einem schlangenlinienförmig gewundenen Stabkörper besteht.

30. Druckbehälter nach einem der Ansprüche 25 bis 29, **dadurch gekennzeichnet, daß** eine Heizung als Wand des Korbes (160) ausgebildet ist.

31. Druckbehälter nach Anspruch 30, **dadurch gekennzeichnet, daß** die der Heizung zugehörigen Steuerelemente auf dem Deckel (144) angebracht sind.

## Claims

1. Pressure container for applying pressure to a flexible bag (36, 76, 160) received in it and filled with a medical fluid (80), in order to deliver the fluid (80) from the flexible bag (36, 76, 160), said container having a housing (12, 52, 142) that has an opening (44) for introduction of the bag (36, 76, 160), and having a cover (14, 54, 144) for covering the opening (44), said cover having an aperture for leading a fluid discharge line (38, 78) of the bag (36, 76, 160) out of the pressure container (10, 50, 140), and said container having a closure (100) for sealed joining of the cover (14, 54, 144) and housing (12, 52, 142),
**characterized in that** the housing (12, 52, 142) is configured as an upright hollow-cylindrical body (16, 56), closed at the bottom and open at the top, the cross section of which has a flattened oval shape and corresponds roughly to the cross-sectional contour of a bag (36, 76, 160); and the two opposing large-area upright sidewalls (20, 22, 60, 62) each have, in mirror-image fashion, the shape of a segment of a cylindrical shell whose lateral ends are joined by joining segments (24, 26; 64, 66).

2. Pressure container of Claim 1, **characterized in that** the cover (14, 54, 144) is configured as a cover (14, 54, 144) which covers the oval upper opening (44) and overlaps the upper edge (28, 68) of the body (16, 56).

3. Pressure container of Claims 1 or 2, **characterized in that** there is provided in at least one sidewall (20, 60) a viewing window (48, 62) whose pane (49, 84) also has the shape of a cylindrical shell segment, and **in that** the pane (49, 84) is placed against the viewing window (48, 82) from the inner side (85) of the housing (52).

4. Pressure container of Claim 3, **characterized in that** at least one circumferentially or axially extending bridge (86) of the material of the sidewall (60) is left behind in the viewing window (82).

5. Pressure container of anyone of Claims 1 through 4, **characterized in that** the closure (100) has at least one turning knob (102), arranged on the outer side of the housing (52), on whose inner side is cut out a gated guide (106) into which a gate peg (114) projecting radially from the cover (54) can be introduced, and **in that** rotation of the turning knob (102), the gate sealingly prevents removal of the cover (54) from the housing (52).

6. Pressure container of Claim 5, **characterized in that** the gate peg (114) has a cylindrical shoulder segment (118), projecting radially from the cover (54), adjoining which is an end equipped with a roller (116) or a needle bearing, the gated guide (106) being configured such that in a first rotational segment the roller (116) or needle bearing runs over a ramp (119), and only in the end position does an end segment of the gated guide (106) with a stop (112) come to rest against the shoulder segment (118) of the gate peg (114).

7. Pressure container of Claims 5 or 6, **characterized in that** the gated guide (106) is undercut in the region of the stop (112).

8. Pressure container of anyone of Claims 1 through 7, **characterized in that** a circumferential V-seal (88) is provided between the cover (56) and the upper edge (68) of the body (14).

9. Pressure container of anyone of Claims 1 through 8, **characterized in that** two closures (100), diametrically opposite one another and arranged on the joining segments (64, 66) of the cylindrical shell walls, are provided.

10. Pressure container of anyone of Claims 1 through 9, **characterized in that** at least one connector (41, 128) for delivering a pressure medium into the pressure container (10, 50) is provided in the cover (14, 54).

11. Pressure container of anyone of Claims 1 through 10, **characterized in that** the inner wall (85) of the housing (12, 52) is equipped with a low-friction lining (87).

12. Pressure container of anyone of Claims 1 through 11, **characterized in that** the pressure container (10, 50) can be mounted on a stand (32, 72).

13. Pressure container of Claim 12, **characterized in that** the housing (12, 52, 142) is joined to the cover (54, 144) in lossproof fashion via an apparatus (120, 145) that can be extended and retracted along the stand axis.

14. Pressure container of Claim 13, **characterized in that** the apparatus (120, 145) is of telescoping configuration.

15. Pressure container of anyone of Claims 12 through 14,
**characterized in that** the cover (14, 54) can be joined immovably to the stand (32, 72), and **in that** the housing (12, 52) is displaceable along the stand axis relative to the cover (14, 54).

16. Pressure container of anyone of Claims 13 through 15,
**characterized in that** there is provided in the apparatus (120) a load-relieving spring (126) which facilitates the operation of displacing the housing (12, 54).

17. Pressure container of Claim 16, **characterized in that** the load-relieving spring (126) comprises a gas spring, a scroll spring, or a spring made of a shape-memory alloy.

18. Pressure container of anyone of Claims 1 through 17, **characterized in that** a hooking apparatus (34, 74) for a bag (36, 76, 160) is provided on the underside of the cover (14, 54, 144).

19. Pressure container of anyone of Claims 1 through 18, **characterized in that** there is provided in the upper edge (28, 68) of the housing (12, 52, 142) at least one cutout (30, 70, 159) into which the fluid discharge line (38, 78) of the bag (36, 76, 160) can be set from above and thereby guided out of the housing (12, 52, 142).

20. Pressure container of Claim 19, **characterized in that** a shaped seal (90), into which the fluid discharge line (38, 78) can be placed, can be set into the at least one cutout (30, 70).

21. Pressure container of Claim 20, **characterized in that** a segment (55) of the cover (52) which overlaps the upper edge (28, 68) of the housing (12, 52) also partially covers the shaped seal (90).

22. Pressure container of Claims 20 or 21, **characterized in that** the shaped seal (90) overlaps the housing wall (60) internally and externally in the region of the cutout (70).

23. Pressure container of anyone of Claims 20 through 22, **characterized in that** the shaped seal (90) is slotted so that the fluid discharge line (78) can be pushed into the shaped seal (90).

24. Pressure container of anyone of Claims 1 through 23, **characterized in that** a heater is provided on the housing (12, 52).

25. Pressure container of anyone of Claims 1 through 24, **characterized in that** a basket (146) into which the bag (160) can be placed is mounted on the cover (144).

26. Pressure container of Claim 25, **characterized in that** the basket (146) has a bottom (152) which comes to rest at a distance above the closed bottom of the housing (152).

27. Pressure container of Claims 25 or 26, **characterized in that** the basket (146) has a guide for the fluid discharge line of the bag (160).

28. Pressure container of Claim 27, **characterized in that** the guide for the fluid discharge line has hooks (156, 157, 158) into which the fluid discharge line can be introduced.

29. Pressure container of anyone of Claims 26 through 28, **characterized in that** the bottom (160) is made of a rod element bent in serpentine fashion.

30. Pressure container of anyone of Claims 25 through 29, **characterized in that** a heater is configured as a wall of the basket (160).

31. Pressure container of Claim 30, **characterized in that** the control elements pertinent to the heater are mounted on the cover (144).

## Revendications

1. Récipient sous pression pour mettre sous pression un sachet souple (36, 76, 160) rempli d'un liquide médical (80) contenu dans celui-ci, afin de distribuer le liquide (80) hors du sachet souple (36, 76, 160), comportant un boîtier (12, 52, 142) qui présente une ouverture (44) pour l'introduction du sachet (36, 76, 160), comportant un couvercle (14, 54, 144) pour recouvrir l'ouverture (44), comportant un ajour pour le départ d'un conduit d'évacuation de liquide (38, 78) du sachet (36, 76, 160) à l'extérieur du récipient sous pression (10, 50, 140), et comportant une fermeture (100) pour la liaison étanche du couvercle (14, 54, 144) et du boîtier (12, 52, 142), **caractérisé en ce que** le boîtier (12, 52, 142) est conformé en corps (16, 56) debout cylindrique creux fermé côté fond et ouvert à sa partie supérieure, dont la section transversale est de forme ovale plate, et qui correspond grossièrement au contour de la section transversale d'un sachet (36, 76, 160), et **en ce que** les deux parois latérales debout (20, 22, 60, 62) de grande surface, se faisant face l'une l'autre, présentent, dans une symétrie en miroir, la forme d'une partie d'une paroi cylindrique dont les extrémités latérales sont reliées par des portions de liaison (24, 26; 64, 66).

2. Récipient sous pression selon la revendication 1, **caractérisé en ce que** le couvercle (14, 54, 144) est conformé en couvercle coiffant (14, 54, 144) recouvrant l'ouverture supérieure (44) ovale et passant sur le bord supérieur (28, 68) du corps (16, 56).

3. Récipient sous pression selon la revendication 1 ou 2, **caractérisé en ce qu'**il est prévu, au moins dans une paroi ovale (20, 60), une fenêtre (48, 82) dont la vitre (49, 84) présente également la forme d'une partie d'enveloppe cylindrique, et **en ce que** la vitre (49, 84) s'applique, depuis le côté intérieur (85) du boîtier (52), contre la fenêtre (48, 82).

4. Récipient sous pression selon la revendication 3, **caractérisé en ce que** dans la fenêtre (82) subsiste au moins une entretoise de matière (86), s'étendant périphériquement ou axialement, de la matière de la paroi latérale (60).

5. Récipient sous pression selon l'une des revendications 1 à 4, **caractérisé en ce que** la fermeture (100) comporte au moins un bouton tournant (102) disposé sur la face extérieure du boîtier (52), sur la face intérieure duquel est découpé un guide-coulisse (106) dans lequel peut être introduite une tige formant coulisse (114) dépassant radialement du couvercle (54), et **en ce que** par rotation du bouton tournant (102), la coulisse bloque de manière étanche le couvercle (54) contre un enlèvement du boîtier (52).

6. Récipient sous pression selon la revendication 5, **caractérisé en ce que** la tige formant coulisse (114) comporte une portion d'épaulement (118) cylindrique faisant saillie radialement du couvercle (54), à laquelle fait suite une extrémité pourvue d'un galet (116) ou d'un roulement à aiguilles, le guide-coulisse (106) étant conçu de manière que dans une première portion de rotation, le galet (116) ou le roulement à aiguilles passe sur une rampe (119), et de manière que dans la position terminale seulement, une portion terminale du guide-coulisse (106) avec butée (112) parvient sur la portion d'épaulement (118) de la tige formant coulisse (114).

7. Récipient sous pression selon la revendication 5 ou 6, **caractérisé en ce que** le guide-coulisse (106) est détalonné dans la zone de la butée (112).

8. Récipient sous pression selon l'une des revendications 1 à 7, **caractérisé en ce qu'**il est prévu une garniture d'étanchéité en V (88) périphérique, entre le bord supérieur (68) du corps (56) et le couvercle (14).

9. Récipient sous pression selon l'une des revendications 1 à 8, **caractérisé en ce que** deux fermetures (100) diamétralement opposées sont prévues sur les portions de liaison (64, 66) des parois de l'enveloppe cylindrique.

10. Récipient sous pression selon l'une des revendications 1 à 9, **caractérisé en ce que** dans le couvercle (14, 54) est prévu au moins un raccordement (41, 128) pour l'amenée d'un fluide sous pression dans le récipient sous pression (10, 50).

11. Récipient sous pression selon l'une des revendications 1 à 10, **caractérisé en ce que** la paroi intérieure (85) du boîtier (12, 52) est pourvue d'une garniture de glissement (87).

12. Récipient sous pression selon l'une des revendications 1 à 11, **caractérisé en ce que** le récipient sous pression (10, 50) peut être placé sur un pied (32, 72).

13. Récipient sous pression selon la revendication 12, **caractérisé en ce que** le boîtier (12, 52, 142) est relié de manière imperdable au couvercle (54, 144), au moyen d'un dispositif (120, 145) qui peut être introduit et extrait le long de l'axe du pied.

14. Récipient sous pression selon la revendication 13, **caractérisé en ce que** le dispositif (120, 145) est réalisé de façon télescopique.

15. Récipient sous pression selon l'une des revendications 12 à 14, **caractérisé en ce que** le couvercle (14, 54) peut être relié fixement au pied (32, 72) et **en ce que** le boîtier (12, 52) est déplaçable par rapport au couvercle (14, 54), le long de l'axe du pied.

16. Récipient sous pression selon l'une des revendications 13 à 15, **caractérisé en ce que** dans le dispositif (120) est prévu un ressort de décharge (126) qui facilite l'opération de déplacement du boîtier (12, 54).

17. Récipient sous pression selon la revendication 16, **caractérisé en ce que** le ressort de décharge (126) est constitué d'un ressort à gaz, d'un ressort à rouleaux ou d'un ressort constitué d'un alliage à mémoire.

18. Récipient sous pression selon l'une des revendications 1 à 17, **caractérisé en ce que** sur la face inférieure du couvercle (14, 54, 144) est prévu un dispositif d'accrochage (34, 74) pour un sachet (36, 76, 160).

19. Récipient sous pression selon l'une des revendications 1 à 18, **caractérisé en ce que** dans le bord supérieur (28, 68) du boîtier (12, 52, 142) est prévu au moins un évidement (30, 70, 159) dans lequel peut être mis en place depuis le haut le conduit d'évacuation de liquide (38, 78) du sachet (36, 76, 160), qui est ainsi guidée vers l'extérieur du boîtier (12, 52, 142).

20. Récipient sous pression selon la revendication 19, **caractérisé en ce que** dans le ou les évidements (30, 70) peut être insérée une garniture d'étanchéité profilée (90) dans laquelle peut être mis en place le conduit d'évacuation de liquide (38, 78).

21. Récipient sous pression selon la revendication 20, **caractérisé en ce qu'**une portion (55) du couvercle (52), coiffant le bord supérieur (28, 68) du boîtier (12, 52), recouvre aussi en partie la garniture d'étanchéité profilée (90).

22. Récipient sous pression selon la revendication 20 ou 21, **caractérisé en ce que** la garniture d'étanchéité profilée (90) passe à l'intérieur et à l'extérieur sur la paroi (60) du boîtier dans la zone de l'évidement (70).

23. Récipient sous pression selon l'une des revendications 20 à 22, **caractérisé en ce que** la garniture d'étanchéité profilée (90) est fendue, ce qui fait que le conduit d'évacuation de liquide (78) peut être enfoncé dans la garniture d'étanchéité profilée (90).

24. Récipient sous pression selon l'une des revendications 1 à 23, **caractérisé en ce qu'**un chauffage est prévu sur le boîtier (12, 52).

25. Récipient sous pression selon l'une des revendications 1 à 24, **caractérisé en ce que** sur le couvercle (144) peut être fixée une corbeille (146) dans laquelle le sachet (160) peut être placé.

26. Récipient sous pression selon la revendication 25, **caractérisé en ce que** la corbeille (146) présente un fond (152) qui vient se placer à distance au-dessus du fond fermé du boîtier (142).

27. Récipient sous pression selon la revendication 25 ou 26, **caractérisé en ce que** la corbeille (146) présente un guide pour le conduit d'évacuation de liquide du sachet (160).

28. Récipient sous pression selon la revendication 27, **caractérisé en ce que** le guide pour le conduit d'évacuation de liquide comporte des crochets (156, 157, 158) dans lesquels peut être introduit le conduit d'évacuation de liquide.

29. Récipient sous pression selon l'une des revendications 26 à 28, **caractérisé en ce que** le fond (152) est constitué d'un corps à barreaux enroulé en serpentin.

30. Récipient sous pression selon l'une des revendications 25 à 29, **caractérisé en ce qu'**un chauffage est réalisé de manière à former la paroi de la corbeille (146).

31. Récipient sous pression selon la revendication 30, **caractérisé en ce que** les éléments de commande qui font partie du chauffage sont placés sur le couvercle (144).
